# EUROPEAN PATENT APPLICATION

(11) **EP 1 203 958 A2**
(43) Date of publication of application: **08.05.2002**
(21) Application number: 01125427.3
(22) Date of filing: 31.10.2001
(51) Int. Cl.: G01N 33/68, G01N 33/543, G01N 33/573, A61P 9/00, C07K 1/22

(54) **High-throughput screening method**

(30) Priority: 01.11.2000 JP 2000334774
(71) Applicant: SUMITOMO PHARMACEUTICALS COMPANY, LIMITED, Osaka-shi Osaka-fu (JP)
(72) Inventor: Imanishi, Noriaki, Ibaraki-shi, Osaka 567-0046 (JP); Kawane, Kenji, Osaka-shi, Osaka 546-0003 (JP); Goda, Masao, Nishinomiya-shi, Hyogo 662-0831 (JP)
(74) Representative: VOSSIUS & PARTNER

(57) **Abstract**

The present invention provides a new screening method. The invention comprises selecting an antibody reactive with only an enzyme that is unstable and difficult to determine, purifying the enzyme from a crude extract in one operation without affecting its activity, and immobilizing the same onto a plate.

## Description

### TECHNICAL FIELD

The present invention relates to a high-performance, efficient screening system for discovering inhibitors or activators of protein functions, or regulators in living cells. The invention also relates to a method of purifying unstable proteins. Specifically, it relates to a high-performance, efficient screening system for discovering inhibitors or activators of serine/threonine kinases represented by Rho-kinase, or regulators in living cells. More specifically, it relates to a screening system for discovering activators or inhibitors of Rho-kinase, or regulators in living cells, which comprises using the kinase in a tissue homogenate, and to instruments useful for the screening system.

### BACKGROUND ART

In order to discover inhibitors/activators of protein functions or regulators in living cells, it is generally necessary to prepare a large amount of an intended protein, and to establish a high-performance, efficient assay system therefor. However, it is not always necessary to prepare a large amount of an intended protein when the protein is responsible for a specific reaction in cells or tissues. In the latter case, the activity of the protein may be specifically detected in a crude extract such as a cell homogenate and a tissue homogenate.

On the other hand, when inhibitors/activators are discovered of a certain subtype of proteins that consist of a family comprising many subtypes having a similar activity said subtype among the family exhibiting its distinctive activity, the preparation of a large amount of the subtype protein and the subsequent preparation of highly purified products by means of for example recombinant technology are necessary. Such proteins are exemplified by enzymes such as kinases (Knighton, D.R. et al., *Science,* 253, 407, 1991; Hanks, S.K., and Hunter, T., *FASEB J.* 9, 576, 1995), phosphatases, (Cohen, P., *Annu. Rev. Biochem.* 58, 453, 1989; Murray, K.J. et al., *Annu. Report Med. Chem.* 29, 255, 1994; Mumby, M.C., and Walter, G., *Physiol. Rev.* 73, 673, 1993; Hubbard, M.J., and Cohen, P., *Trends Biochem. Sci.* 18, 172, 1993; Chen, J. et al., *J. Biol. Chem.* 269, 7957, 1994; Mauro, L.J., and Dixon, J.E., *Trends Biochem. Sci.* 19, 151, 1994), esterases, and lipases (Kriz, R. et al., *Ciba Found. Symp*., 150, 112-127, 1990). Further, in case that a crude extract contains any interfering substance of the activity of a subject enzyme in an assay system, it is difficult to discover inhibitors/activators of the certain enzyme since the reproducibility of the assay is reduced, and therefore in such case it is also necessary to prepare a large amount of an intended enzyme, and to further prepare highly purified products, similarly to the above cases.

In order to prepare a highly purified enzyme from crude tissue extracts, it is generally necessary to prepare an amount of material several decades to several hundreds times as large as the intended amount to be assayed, and to conduct chromatography on the material using many kinds of columns, all of which would require troublesome and time-consuming tasks. During the procedures, enzymes are often inactivated, and the purified enzyme may be insufficiently isolated, finally leading to the failure of the purification. Although affinity chromatography can be advantageously used for enzyme purification, it may have a trouble when enzymes captured on the column conjugated with antibodies are dissolved therefrom. Specifically, when enzymes are bound to the antibody-conjugated column with a strong affinity based on antigen-antibody reactions in affinity chromatography, those enzymes are rarely diluted without inactivation.

In case that a highly purified enzyme is prepared from the genetic recombinants, it is necessary to clone the gene, to check whether the gene sequence encodes an intended enzyme, to transform the sequence into host cells, and to purify the enzyme from the cell culture, all of which procedures also would require troublesome and time-consuming tasks.

### SUMMARY OF THE INVENTION

The present invention aims to provide a screening method useful in an assay wherein the isolation of a protein used in the screening method is difficult since the purification of that protein requires the troublesome and time-consuming tasks. Particularly, it aims to provide a high-performance screening system for unstable proteins, of which activities are difficult to determine in crude extracts of cell or tissue homogenates, which comprises selecting antibodies reactive to only the protein, purifying the protein from the crude extract in one operation without inactivation of the protein function, and immobilizing the protein onto a plate.

The present method screens various ones that are soluble or potentially soluble, and, previously, any method enabling to efficiently screen proteins that are difficult to isolate in a large amount due to their instability as described above have not been found.

Thus, the present inventors focused on Rho-kinase as one of those proteins, and attempted to construct a screening system which comprises using the protein.

Rho-kinase was recently discovered and identified as a serine/threonine kinase, which interacts with RhoA that is an Rho family member (Matsui T. et al., *EMBO. J.* 15, 2208-2216, 1996; Ishizaki, T. et al., *EMBO J.* 15, 1885-1893, 1996). The enzyme is distributed throughout vascular smooth muscle similarly to Rho, and phosphorylates myosin light chain (MLC). The enzyme also phosphorylates a myosin-dephosphorylating enzyme, and inhibits the activity of the latter enzyme (Amano, M. et al., *Science* 275, 1308-1311, 1997; Kimura, K. et al., *Science* 273, 245-248, 1996). Like this, Rho-kinase induces the MLC phosphorylation level increased, and is believed to cause smooth muscle contraction.

Agents that block the Rho-kinase functions are believed useful for treating diseases such as hypertension, cardiac angina, and cerebrovascular spasm, because smooth muscle contraction is deeply involved in these diseases. In fact, it has been reported that Y-27632 that is an inhibitor of Rho-kinase exhibits an antihypertensive action in animal models for hypertension such as SHR, a rat model for renal hypertension, DOCA-Salt rat, whereas it exhibits no antihypertensive action in nomal Wistar rat (Uehera M. et al., *Nature* 389, 990-994, 1997). Although Fasudil (Product Name: ERIL, Asahi Kasei Corporation) that has been marketed as a medicament for treating cerebrovascular spasm was known as a MLC kinase inhibitor, it has been shown that it exhibits a stronger inhibitory action on Rho-kinase than MLC kinase (Uehera M. et al., *Nature* 389, 990-994, 1997).

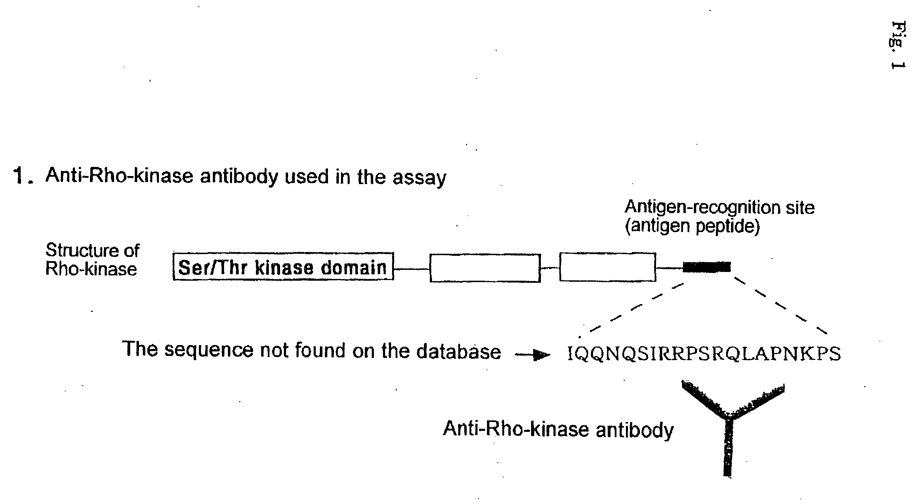

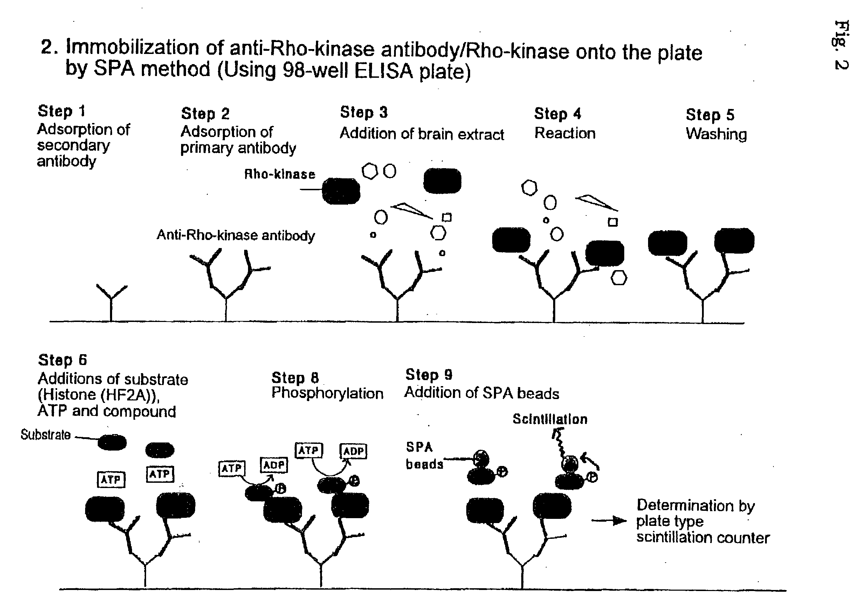

In case of Rho-kinase, for example, the present invention makes it possible to both isolate the enzyme from crude extracts and to construct the assay system in one operation comprising the steps shown in Figures 1 and 2.

Thus, the present invention relates to:
(1) A method of screening for an activator or an inhibitor of the protein function of a soluble protein or a potentially soluble protein, which comprises:
   1) preparing a partial peptide comprising a part of the amino acid sequence of the protein, said partial peptide having the following properties;
      i) the partial peptide comprises an amino acid sequence characteristic of the protein and distinct from proteins belonging to the same family as the protein;
      ii) the amino acid sequence comprises at least six amino acid residues, and is located in a highly hydrophilic region;
      iii) the amino acid sequence has a functional group capable of binding to a carrier protein; and
      iv) the protein function is not inactivated by the reaction between the protein and an antibody raised using the partial peptide as an antigen;
   2) preparing an antibody having an affinity of 10⁵/M or less (Ig), by use of the partial peptide as an antigen;
   3) preparing a solid support immobilized with an antibody to the antibody (Ig) prepared in step 2;
   4) immobilizing the antibody (Ig) prepared in step 2 on the solid support prepared in step 3;
   5) applying a cell or tissue homogenate containing the protein to the solid support prepared in step 4 to immobilize the protein thereon;
   6) reacting a solution of a test substance with the solid support immobilized with the protein as prepared in step 5; and
   7) measuring the activity of the protein after completion of the reaction, so as to determine the effect of the test substance on the protein function.
(2) The method of the above (1), wherein the soluble protein or the potentially soluble protein is a solubilized enzyme.
(3) The method of the above (2), wherein the solubilized enzyme is a serine/threonine kinase.
(4) The method of the above (1), wherein the protein function of the soluble protein or the potentially soluble protein is determined by the incorporation of ³²P or ³³P into a substrate, which is estimated using radioactivity of the phosphorus or binding activity of an antibody to a phosphorylated substrate.
(5) The method of the above (4), wherein the protein function is determined by the effect of the test substance, which is estimated using:
   a) SPA (Scintillation Proximity Assay) method,
   b) Multiscreen method, or
   c) a filter-spot method.
(6) The method of the above (3), wherein the serine/threonine kinase is Rho-kinase.
(7) The method of the above (6), wherein Rho-kinase is from bovine, mouse, or rat.
(8) The method of any one of the above (1) to (7), wherein the tissue homogenate is from bovine cerebral gray matter.
(9) The method of any one of the above (1) to (8), wherein the soluble protein is Rho-kinase, and the partial peptide comprises 20 amino acid residues resided at the C-terminal part of Rho-kinase.
(10) The method of any one of the above (1) to (9), wherein the partial peptide comprises the 20 amino acid residues of IQQNQSIRRPSRQLAPNKPS.
(11) An activator or an inhibitor of the protein function, which is obtainable by conducting the method of any one of the above (1) to (10).
(12). The activator or the inhibitor of the above (11), wherein the protein is Rho-kinase.
(13) The activator or the inhibitor of the above (12), wherein the protein is bovine Rho-kinase.
(14) A pharmaceutical composition, which comprises the activator or the inhibitor of any one of the above (11) to (13) as an active ingredient in admixture with a pharmaceutically acceptable excipient.
(15) A pharmaceutical composition for inhibiting phosphorylation, which comprises the inhibitor of Rho-kinase of the above (12) as an active ingredient in admixture with a pharmaceutically acceptable excipient.
(16) A pharmaceutical composition for inhibiting smooth muscle contraction, which comprises the inhibitor of Rho-kinase of the above (12) as an active ingredient in admixture with a pharmaceutically acceptable excipient.
(17) A pharmaceutical composition for treating hypertension, cardiac angina, or cerebrovascular spasm, which comprises the inhibitor of Rho-kinase of the above (12) as an active ingredient in admixture with a pharmaceutically acceptable excipient.
(18). A method of treating hypertension, cardiac angina, or cerebrovascular spasm, which comprises administering a patient having or at risk of developing one of the diseases an effective amount of the composition of the above (17).
(19). A pharmaceutical composition for promoting phosphorylation, which comprises the activator of Rho-kinase of the above (12) as an active ingredient in admixture with a pharmaceutically acceptable excipient.
(20) A pharmaceutical composition for inducing smooth muscle contraction, which comprises the activator of Rho-kinase of the above (12) as an active ingredient in admixture with a pharmaceutically acceptable excipient.
(21) A method of purifying a protein, which comprises:
   1) preparing a partial peptide comprising a part of the amino acid sequence of the protein, said partial peptide having the following properties;
      i) the partial peptide comprises an amino acid sequence characteristic of the protein and distinct from proteins belonging to the same family as the protein;
      ii) the amino acid sequence comprises at least six amino acid residues, and is located in a highly hydrophilic region;
      iii) the amino acid sequence has a functional group capable of binding to a carrier protein; and
      iv) the protein is not inactivated by the reaction between the protein and an antibody raised using the partial peptide as an antigen;
   2) preparing an antibody having an affinity of 10⁵/M or less (Ig), by use of the partial peptide as an antigen;
   3) preparing a solid support immobilized with an antibody to the antibody (Ig) prepared in step 2;
   4) immobilizing the antibody (Ig) prepared in step 2 on the solid support prepared in step 3;
   5) applying a cell or tissue homogenate containing the protein to the solid support prepared in step 4 to immobilize the protein thereon; and
   6) releasing and recovering the protein from the solid support.
(22) The method of the above (21), wherein the protein is an unstable protein, a soluble protein or a potentially soluble protein, preferably Rho-kinase.
(23) The method of the above (22), wherein Rho-kinase is derived from bovine, mouse, or rat.
(24) A solid support useful for a purification system of a protein, or a screening system of a soluble protein or a potentially soluble protein, which is prepared by a process comprising:
   1) preparing a partial peptide comprising a part of the amino acid sequence of the protein, said partial peptide having the following properties that;
      i) the partial peptide comprises an amino acid sequence characteristic of the protein and distinct from proteins belonging to the same family as the protein;
      ii) the amino acid sequence comprises at least six amino acid residues, and is located in a highly hydrophilic region;
      iii) the amino acid sequence has a functional group capable of binding to a carrier protein; and
      iv) the protein is not inactivated by the reaction between the protein and an antibody raised using the partial peptide as an antigen;
   2) preparing an antibody having an affinity of 10⁵/M or less (Ig), by use of the partial peptide as an antigen;
   3) preparing a solid support immobilized with an antibody to the antibody (Ig) prepared in step 2; and
   4) immobilizing the antibody (Ig) prepared in step 2 on the solid support prepared in step 3.
(25) The solid support of the above (24), wherein the protein is an unstable protein in the purification system, or the soluble protein or the potentially soluble protein is Rho-kinase in the screening system.
(26) A solid support immobilized with a soluble protein or a potentially soluble protein useful for a screening system, which is prepared by a process comprising:
   1) preparing a partial peptide comprising a part of the amino acid sequence of the protein, said partial peptide having the following properties that;
      i) the partial peptide comprises an amino acid sequence characteristic of the protein and distinct from proteins belonging to the same family as the protein;
      ii) the amino acid sequence comprises at least six amino acid residues, and is located in a highly hydrophilic region;
      iii) the amino acid sequence has a functional group capable of binding to a carrier protein; and
      iv) the protein is not inactivated by the reaction between the protein and an antibody raised using the partial peptide as an antigen;
   2) preparing an antibody having an affinity of 10⁵/M or less (Ig), by use of the partial peptide as an antigen;
   3) preparing a solid support immobilized with an antibody to the antibody (Ig) prepared in step 2;
   4) immobilizing the antibody (Ig) prepared in step 2 on the solid support prepared in step 3; and
   5) applying a cell or tissue homogenate containing the protein to the solid support prepared in step 4 to immobilize the protein thereon.
(27) The solid support of the above (26), wherein the soluble protein or the potentially soluble protein is Rho-kinase, and the support is in the form of plate.
(28) The solid support of the above (27), wherein the tissue homogenate is from the bovine cerebral gray matter, or from the rat brain.
(29) The solid support of the above (27) or (28), wherein Rho-kinase is from bovine.
(30) The method of any one of the above (1) to (10), and (21) to (23), wherein the antibody prepared by use of the partial peptide as an antigen has an affinity of 10⁶/M or less.
(31) The activator or the inhibitor of any one of the above (11) to (13), wherein the antibody prepared by use of the partial peptide as an antigen has an affinity of 10⁶/M or less.
(32) The pharmaceutical composition of any one of the above (14) to (20), wherein the antibody prepared by use of the partial peptide as an antigen has an affinity of 10⁶/M or less.
(33) The solid support of any one of the above (24) to (29), wherein the antibody prepared by use of the partial peptide as an antigen has an affinity of 10⁶/M or less.

### BRIEF DESCRIPTION OF THE DRAWINGS

Figure 1 shows an example of the amino acid sequence and the location of a partial peptide of Rho-kinase used as an antigen peptide for the preparation of an anti-Rho-kinase antibody.
Figure 2 shows reaction schemes depicting the principle of the assay system of the present invention using Rho-kinase as an example.
Figure 3 shows the experimental result of the hydrophobicity of bovine Rho-kinase.
Figure 4 represents the graphs showing that phosphorylations are dependent on the dose of anti-Rho-kinase antibody in the assay system of the present invention.
Figure 5 represents the Western blotting analysis showing that the solid support of the present invention permits to recover Rho-kinase depending on the dose of anti-Rho-kinase antibody.
Figure 6 represents the graphs showing that RhoA affects the Rho-kinase reactivity in the immunoprecipitates.
Figure 7 shows the dose-dependent influence of the secondary antibody in the assay system of the present invention.
Figure 8 represents the graph showing an inhibitory effect of the antigen peptide on immobilization of Rho-kinase.
Figure 9 represents the graphs showing that the Rho-kinase inhibitors inhibit Rho-kinase in a dose-dependent manner in the screening system of the present invention.

### DETAILED DESCRIPTION OF THE PREFERRED EMBODIMENTS

In the first aspect, the present invention provides a method of screening for an activator or an inhibitor of the protein function of a soluble protein or a potentially soluble protein (hereinafter, it may be abbreviated as the present screening method).

"Soluble protein(s)" as used in the present invention includes an enzyme, a transcription factor, a nuclear receptor (a proteinous factor required in transcription reactions except for RNA polymerase, which binds to extracellular ligands to exert an action on the transcription), an adaptor protein, an secreted protein, a biologically active substance. Transcription factors include for example Sp1, AP1, and NF-_{κ}B; nuclear receptors include for example a steroid receptor, and PPARa; adaptor proteins (a protein which exhibits no enzyme activity and comprises a region to be bound to other proteins) include for example PLCg and rasGAP having the SH2 domain, and Grb-2 having the SH3 domain; and secreted proteins and biologically active substances include for example a growth factor, insulin, a cytokine, and a chemokine.

Enzymes are preferably those present in the soluble fractions of intra- and extra-cellular compartmentations, and include a serane/threonine kinase, some kinds of tyrosine kinases, an esterase, a protease, and some kinds of phosphatases.

In the invention, "potentially soluble protein(s)" means an enzyme present in the membrane fractions, or a transmembrane receptor, which is a molecule present in the membrane fractions and which may be solubilized by limited proteolysis with surfactants or proteases, or by phospholipase treatment. The proteins include for example CD4, selectin, CD14, glycosyltransferase, and some kinds of receptors, and galactosyltransferase, CD4, and selectin are preferred.

In the invention, the term "serine/threonine kinase" is a general name of enzymes that phosphorylate the hydroxyl group on the serine or threonine residue of a protein. For example, the kinases include Rho-kinase, Protein kinase C, and Protein kinase A, and Rho-kinase is preferred.

In the present invention, "Rho-kinase" means a serine/threonine kinase that interacts with a Rho family member, Rho A. Not only the human type of the enzyme but also the enzymes from other species such as bovine and mouse can be used in the present invention. The amino acid sequence and the base sequence of mouse type of Rho-kinase are described in Nakagawa, O., Fujisawa, K., Ishizaki, T., Saito, Y., Nakao, K. and Narumiya, S., *FEBS Lett.* 392(2), 189-193 (1966), and can be searched under Genbank accession Nos. NM 009071 and NM 09072. The amino acid sequence and the base sequence of rat type of Rho-kinase are described in Leuting, T., Manser, E. Tan,L., and Lim, L., *J. Biol. Chem.* 270 (49), 29051-29054 (1995), and can be searched under Genbank accession Nos. U38481 and U61266. The amino acid sequence and the base sequence of bovine type of Rho-kinase are described in Matsui, T., Amano, M., Yamamoto, T., Chihara, K., Nakafuku, M., Ito, M., Nakano, T., Okawa, K., Iwamatsu, A and Kaibuchi, K, *EMBO J.* 15(9), 2208-2216 (1996), and can be searched under Genbank accession No. U36909. The amino acid sequence and the base sequence of human type of Rho-kinase are described in Narumiya, S. and Iwamatsu, A, Patent: JP 1997135683-A 1 27-May-1997, Takahashi, N., Tuiki, H., Saya, H. and Kaibuchi, K., *Genomics* 55(2), 235-237 (1999), and can be searched under Genbank accession Nos. E13124, D87931.

"Partial peptide" used as an antigen in the present invention comprises a domain in a soluble protein or a potentially soluble protein, which domain is far from a region responsible for the protein function, and which domain comprises the amino acid sequence which is in a non-conserved region and is characteristic of the protein among the amino acid sequences of the protein family members, and which domain comprises at least six amino acid residues, and is located in a highly hydrophilic region. Further, the amino acid sequence of the domain has a functional group capable of binding to a carrier protein. It should be noted that the protein function is not inactivated by the reaction between an antibody raised using the partial peptide as an antigen and the protein.

In this context, the partial peptide comprising the amino acid sequence corresponding to the above properties may be obtained in accordance with well-known methods or methods pursuant thereto. For example, the peptide may be obtained consulting "Antibodies - A laboratory manual", (p72-87, Harlow & Lane, 1988, Cold Spring Harbor Lab).

In case that the protein is of serine/threonine kinase series, for example, a partial peptide which is in a non-conserved region, and which comprises at least six amino acid residues may be selected from amino acid sequences far from the region responsible for the protein activity.

In case that the protein is Rho-kinase, the following example for obtaining a partial peptide of Rho-kinase may be proposed. Taking account of the primary structure of Rho-kinase comprising the kinase domain located at the N-terminal side, the coiled coil domain located at the central part, and the PH domain located at the C-terminal side, a region which is located near the C-terminal and which is characteristic of Rho-kinase (antigen peptide, 20 amino acid residues) is selected in order to not cause the steric hindrance of the enzyme activity-responsible domain located near the N-terminal. Then, an antigen is prepared based on the amino acid sequence of the selected region, and used to prepare polyclonal antibodies such as rabbit Ig.

For example, IQQNQSIRRPSRQLAPNKPS may be exemplified as a region comprising the amino acid sequence characteristic of Rho-kinase (antigen peptide, 20 amino acid residues).

In cases of other enzymes, suitable antigen peptide can be also obtained in a similar matter.

In the present invention, "highly hydrophilic region" means a region that exhibits a high affinity for the water molecule, and is composed of the hydrophilic amino acids such as lysine, arginine, glutamic acid, and aspartic acid. In cases of soluble proteins and potentially soluble proteins, the region is generally positioned outside the protein molecules, and therefore the region constitutes epitope in the proteins.

In the present invention, "functional group capable of binding to a carrier protein" means the SH group of cysteine, the hydroxyl group of tyrosine, the carboxyl group of glutamic acid or aspartic acid, and the amino group of lysine. Suitable number of these functional groups in the partial peptide makes it possible to bind the partial peptide to a carrier protein. Accordingly, the amino acid sequence of the partial peptide necessarily contains sufficient number of the functional groups to bind to a carrier protein, and preferably contains many functional groups.

In the present invention, "an antibody to an antibody (Ig) prepared by use of the partial peptide as an antigen" mean an antibody designed to not damage the binding activity of the antibody prepared by use of the partial peptide to the antigen. In general, the former is called secondary antibody, whereas the latter is called primary antibody. Secondary antibody is preferably prepared using as a antigen the Fc region of the corresponding isotype from the same animal species as those of the primary antibody. It is recommendable that the whole region, the Fab, or F(ab')₂ should not be used, because they may affect the activity of the primary antibody.

Primary antibody and secondary antibody as shown above may be a polyclonal or monoclonal antibody.

Affinity of the antibodies in the present invention is preferably 10⁵/M or less, and more preferably 10⁶/M or less.

In the present invention, "cell homogenate or tissue homogenate" means a suspension obtained by destroying cells or tissues in a suitable buffer with a homogenizer. Kinds of the cell homogenate or the tissue homogenate may be selected as appropriate, depending on the purpose of the screening. For example, tissue homogenates derived from bovine cerebral gray matter, or from rat brain can be used in case of Rho-kinase. In case of enzymes expressed in a tissue-specific manner, the homogenate derived from the tissue where the enzymes are expressed in a tissue-specific manner may be used to establish the screening system.

In the present invention, "applying a cell or tissue homogenate containing the protein to the solid support to immobilize the protein thereon" means that the protein is bound to a solid support such as a plate or resin beads via the antibody shown above so that the protein is hard to release from the support during the normal washing procedures.

In the present invention, "reacting a solution of a test substance with the solid support" means that a substance expected to exhibit a property of an inhibitor or an activator, and an enzyme on which the substance affect are mixed and reacted together so as to prepare for an activity determination.

In the invention, "measuring the activity of the protein" means that the quantity of a suitable substrate converted by the protein under an optimal condition is determined. Enzyme activity is expressed as a unit, and is generally defined as amount of an enzyme that converts one mol of a.substrate at 30 °C per minute under an optimal condition for the enzyme. The definition of a unit can be changed, when a substrate has a higher molecular weight, or is hard to express in molar unit.

As described below, the solid support adherently bound with the protein can be directly used in a screening method of the present invention. For example, the present invention may be usually conducted by the two manners of the following. One manner comprises immobilizing the protein onto a microplate such as a 96-well plate, and performing on the immobilized plate all steps through the determination of the activity, likely in SPA (Scintillation Proximity Assay) method as shown below, and the other manner comprises proceeding the reaction of the protein function in the wells of the plate, transferring the solution into anther plate or filter after completion of the reaction, and performing the necessary steps therein to determine the activity, likely in Multiscreen method, or a filter-spot method.

A screening method of the present invention using Rho-kinase as a soluble protein may be conducted for example by the following method comprising the screening methods as shown above.

First of all, a test substance is reacted with a solid plate bound to Rho-kinase, and a substrate for Rho-kinase is added thereto. Then, the substrate is allowed to react with Rho-kinase, and the extent of the phosphorylation inhibition of the substrate is used as an indicator to check if the test substance is an inhibitor or an activator of Rho-kinase. The screening method may be specifically described by the following three manners.
1) SPA method
   i) By use of streptavidin-PVT beads
      To the plate immobilized with an enzyme, 30 µl of a reaction (20Mm Tris-Cl (pH7.5), 10mM MgCl₂, 0.1mM ATP, 1.85KBq[³²P]-ATP) and biotin-labeled Histone HF2A at 0.2mg/ml as a substrate are added. After completion of the reaction, a quenching solution is added thereto. The quenching solution is composed of 500 µM ATP, 20mM EDTA, 0.1% BSA, and 0.1mg streptavidin-PVT beads. After the reaction mixture is allowed to stand for five hours, the determination is conducted with MicroBeta (Wallac).
   ii) By use of poly-L-lysine-YSi beads (SPA-YSi method)
      As a substrate, biotin-labeled Histone HF2A is used at 0.2mg/ml. After completion of the reaction, a quenching solution is added thereto. The quenching solution is composed of 500 µM ATP, 20mM EDTA, 0.25mg poly-L-lysine-YSi beads/150 µl. After the reaction mixture is allowed to stand for two hours, the determination is conducted with MicroBeta (Wallac).
2) Multiscreen method
   i) TCA precipitation - PVDF membrane
      After completion of the reaction, EDTA is added to the reaction at the final concentration of 30 mM, and the mixture is added to Multiscreen HV (Millipore, MHVB N45) that have been added with 50 µl of 20 mM ATP and 100 µl of an ice-cooled 30% TCA (trichloroacetic acid). One hundred µl of 30% TCA is poured onto this, and the material is allowed to stand at 4 °C for 30 minutes. Then, the material is filtered with suction, and washed three times with 100 µl of 30% TCA with suction. Further, it is washed with 100 µl of 100% ethanol, and sucked dry. To this material, 20 µl of a scintillation cocktail (ACS-II) is added, and the fluorescence is determined with MicroBeta (Wallac).
   ii) TCA precipitation - cation exchange phosphocellulose membrane
      After completion of the reaction, phosphoric acid at the final concentration of 75 mM and 25mM ATP are added to the reaction, and the mixture is added to Multiscreen PH (Millipore, MAPH-MOB). After the suction, the material is washed five times with 75mM phosphoric acid with suction, further washed with 100 µl of 100% ethanol, and sucked dry. To this material, 20 µl of a scintillation cocktail (ACS-II) is added, and the fluorescence is determined with MicroBeta (Wallac).
3) filter-spot method
   After completion of the reaction, phosphoric acid at the final concentration of 75 mM, and 500 µM ATP are added to the reaction, and 15 µl portion of the mixture is spotted onto Filter P30 for MicroBeta (Wallac, 1450-523). The material is washed under shaking in a plastic vessel containing 150 ml of 75 mM phosphoric acid (10 minutes x3), and dried in a microwave oven for three minutes. The solid scintillator (MeltiLex, Wallac) is melted, then used to encapsulate the material, and solidified, followed by conducting the determination with MicroBeta (Wallac).

Among the screening methods as shown above, the SPA-YSi method, and the filter-spot method are preferred, with the SPA-YSi method being more preferred.

Especially, a SPA-YSi method wherein a native histone is used as a substrate, and the reaction products are trapped on SPA beads via protein adsorption can provide a good result. In this method, poly-L-lysine-YSi beads that can provide higher counts and are of low cost, are preferably used, although any SPA beads can bind to a protein to permit the detection of the activity.

Relevancy of this method as a screening assay system is proved by the following facts:
i) Background: 1.85 fold or more against the S/N; back, provided that Non Proximity Effect inherent in ³³P can not be eliminated, although this is permitted in an assay;
ii) Dispersion: about 10% of CV in 48-well
iii) Equivalency: Inhibition profiles of a known inhibitor are almost identical between the SPA-YSi method and the filter-spot method.

In the second aspect, the present invention provides an activator or an inhibitor obtainable by conducting the screening method of the present invention.

In the present invention, "an activator or an inhibitor" means a substance inducing an activation or an inhibition of the function of the corresponding protein. In case of Rho-kinase as a protein, for example, activators or inhibitors of Rho-kinase can control the phosphorylation activity of Rho-kinase.

Specifically, the activators of the present invention promote the phosphorylation activity of Rho-kinase, and induce smooth muscle contraction. The inhibitors of the present invention inhibit the phosphorylation activity of Rho-kinase, and inhibit smooth muscle contraction.

Smooth muscle contraction is deeply responsible for the etiology of the diseases such as hypertension, cardiac angina, and cerebrovascular spasm, and the inhibitors of Rho-kinase are believed to be a medicament and/or a prophylactic for these diseases. For example, Rho-kinase inhibitors can be used as an antihypertensive drug.

As shown above, the screening method of the present invention is useful for selecting a medicament and/or a prophylactic for cardiovascular diseases caused by smooth muscle contraction.

Further, the present invention also provides a pharmaceutical composition for inhibiting phosphorylation, which comprises the inhibitor of Rho-kinase as an active ingredient in admixture with a pharmaceutically acceptable excipient.

In the present invention, "inhibitors of Rho-kinase" mean a substance that bind to Rho-kinase to inhibit the phosphorylation of the substrates such as MLC, Myelin basic protein, histone H1, and HF2A.

In the invention, "activators of Rho-kinase" mean a substance that bind to Rho-kinase to activate the phosphorylation of the substrates such as MLC, Myelin basic protein, histone H1, and HF2A.

Methods for administration, dosage forms, and doses for pharmaceutical compositions of the present invention comprising activators or inhibitors of protein functions as active ingredients are described hereinafter.

Pharmaceutical composition comprising low molecular weight compounds, proteins or peptides as activators and/or inhibitors of protein functions of the present invention are formulated into forms commonly known in the art, and are administered orally or parenterally. Dosage form and administration method are generally as shown below.

The composition can be orally administered in dosage form used commonly in the art, and can be parenterally administered as topical (including transdermal application), transrectal, injectable and transnasal formulations.

Oral or transrectal formulations include capsules, tablets, pills, powders, drops, suppositories, solutions, or the like. Injectable formulations include sterile solutions or suspensions, and emulsions, and the specific examples include water, water-propylene glycol solution, buffered solution, 0.4 % physiological saline. Liquefied formulation can be stored in a frozen state, or in a water-free state by lyophilization, etc. The lyophilized formulation may be reconstituted by adding distilled water for injection when used. Topical formulations include creams, ointments, lotions, and transdermal formulations.

Such dosage forms may be prepared in accordance with conventional manners by combining the active ingredient with pharmaceutically acceptable excipients or additives. Those pharmaceutically acceptable excipients or additives include carriers, binders, flavoring agents, buffering agents, thickening agent, coloring agent, stabilizing agents, emulsifying agents, dispersing agents, suspending agents, preservatives, pH regulators, isotonization regulators, spreaders, or the like. Pharmaceutically acceptable carrier include magnesium carbonate, lactose, pectin, starch, and methyl cellulose.

Those pharmaceutical compositions may be administered via a route suitable depending on an intended disease, or a target tissue. For example, the compositions may be administered intraarterially, intravenously, subcutaneously, intradermally or intramuscularly, and administered topically to a diseased tissue directly. Alternatively, the composition can be administered orally or as a suppository.

Doses and frequencies vary depending on the disease and condition to be treated, the age and weight of a particular patient, and the like, and a typical daily dose for adults of the active ingredients may range about 0.0001 mg to about 500 mg, preferably, about 0.001 to about 100 mg, which may be administered at a time or in portions.

In the third aspect, the present invention provides a method of purifying and isolating a protein (hereinafter, it may be abbreviated as the present purification method).

In the present purification method, "releasing and recovering the protein from the solid support" means that the protein immobilized on the solid support is released therefrom and recovered by means of well-known methods such as modifications of the pH value or the salt concentration. For example, an immobilized protein may be efficiently released by use of the solution containing antigen peptide used in the preparation of the antibody, although depending on a binding affinity between the immobilized protein and the antibody thereto.

In case that a protein is Rho-kinase, for example, the protein can be purified by eluting a solution containing a partial peptide used as an antigen through the column immobilized with the protein, and recovering the protein from the effluent, thus obtaining the protein, Rho-kinase in high purity.

Such purification method is useful for purifying unstable proteins such as Rho-kinase, and can provide bovine Rho-kinase in a large amount, and thus highly purified one.

In the forth aspect, the present invention provide a solid support useful for a purification system of a protein or a screening system of a soluble protein or a potentially soluble protein (hereinafter, it may be abbreviated as the present solid support) and a method therefor, as well as a solid support immobilized with a soluble protein or a potentially soluble protein useful for a screening system (hereinafter, it may be abbreviated as the present immobilized solid support).

The present solid support is not limited to particular species as long as the support can be used as conventional carriers. For example, the solid support may be 96-well microplate, beads of resin or the like.

The present solid support may be prepared by immobilizing onto a solid support an antibody (secondary antibody) designed to not destroy the binding activity of an antibody (primary antibody) prepared using a partial peptide of a protein as an antigen.

In case that the protein is Rho-kinase, for example, an anti-Rho-kinase antibody used as a primary antibody is added to a mixture of the rabbit anti-goat IgG/Fcs to select a rabbit anti-goat IgG/Fc that can be bound to the anti-Rho-kinase antibody. Then, the resultant rabbit anti-goat IgG/Fc is used as a secondary antibody to immobilize the anti-Rho-kinase antibody onto a solid support such as a plate via the secondary antibody recognizing the Fc fragment of the primary antibody.

The present invention is further illustrated by the following examples, but is not restricted by these examples in any way.

### Example 1

### Preparation of partial peptides useful for the preparation of anti-Rho-kinase antibodies

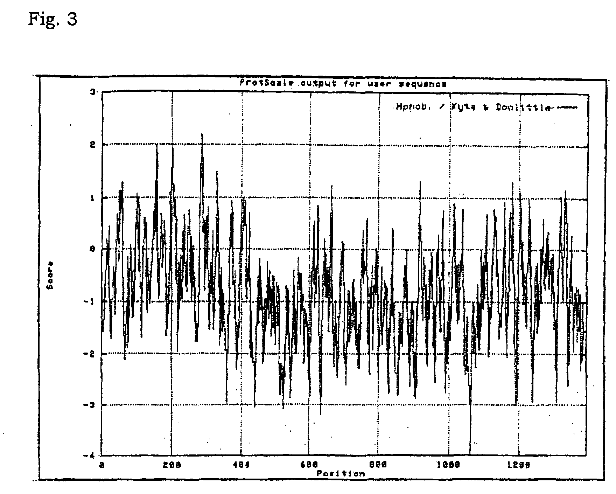

Hydrophobicity analysis of the amino acid sequence of the Rho-kinase derived from the bovine brain was conducted in accordance with Kyte & Doolittle method, revealing that a sequence of a highly enhanced hydrophilicity was found in the C-terminal region, which is opposite to the N-terminal region where the catalytic domain of Rho-kinase is resided (Figure 3). Then, the sequence of 20 amino acids resided near the C-terminal, IQQNQSIRRPSRQLAPNKPS, was selected, and a Blast search was conducted on the sequence using the GenBank database, showing that the sequence shares a homology with Rho-kinase enzymes derived from diverse species (human; GenBank accession Nos: D87931, AB014519), mouse; GenBank accession No: U58513, rat; GenBank accession No: U38481, bovine; GenBank accession No: U36909), and from platanna (GenBank accession No: AF037073), and no homology with any other molecules.

In the light that the sequence contains only one ε-amino group of Lysine, it was believed not so difficult to bind a carrier protein to the amino acid sequence at its N-terminal, and therefore, the sequence was selected as an antigen.

### Example 2

### Preparation of antibody

### A. Binding of the antigen peptide to carrier protein

The antigen peptide as prepared above was dissolved in PBS to give 5 mg/ml solution of the antigen peptide. Employing ovalbumin to be used as a carrier protein, 1 mg/ml solution of the protein was prepared. To 2 ml of the latter solution was added 5 to 10 µl of the antigen peptide solution, and the mixture was thoroughly stirred. To the mixture was added portionwise 0.2 % solution of glutaraldehyde in PBS, and the mixture was stirred for one hour at room temperature. One M solution of glycine in PBS was added thereto at the final concentration of 200 mM, and the mixture was further stirred for one hour. Then, ultrafiltration was conducted on the mixture, or dialysis was repeated against PBS.

### B. Immunization of rabbit with the antigen peptide

For immunization of a rabbit of weight about 2.5kg, 10 mg/ml of the peptide-ovalbumin complex was mixed 1 : 1 with a Freund's complete adjuvant in a tube, and the mixture was thoroughly stirred by sonication for 20 seconds to one minute until the mixture was observed to become a water-in-oil emulsion. To the emulsion was added an equivalent volume of 2% Tween 80-physiological saline, and the mixture was further stirred by sonication for 20 seconds. The mixture was putted into a syringe, and injected subcutaneously into rabbits at several parts (each back side, glutaeus or the like). At each part, about 0.2 ml was injected. Subsequently, a similar injection was conducted three times every two weeks. Two weeks after the final injection, a blood sample was taken from the auricular veins to prepare the blood serum, and antibody titers thereof were checked by ELISA. When the titer was shown to be sufficient, the whole blood was taken from the animals to prepare the blood serums.

### C. Purification of IgG fractions with protein A-Sepharose

The blood serum was diluted twofold with PBS, and the dilution was loaded onto the A-Sepharose CL-4B column. PBS was eluted at a flow rate of 30 ml/hr to sweep fully the proteins that were not bound to the gel, and then the bound IgG was eluted with a 0.1 M glycine hydrochloride buffer (pH2.8). The effluent was neutralized with NaOH, and dialyzed against PBS.

### Example 3

### Estimation of Rho-kinase activity in immunoprecipitates with anti-Rho-kinase antibody

Method and Material
GST-RhoA (Cat. No.555466 CALBIOCHEM)
Bovine brain (Shiraimatsu)
Anti-Rho-kinase peptide antibody derived from goat (Lot: #079, 200 µg/µl Santa Cruz Biotechnology)
Rabbit anti-goat IgG (Fc) antibody (Lot: 19030542, 2.4 mg/ml CHEMICON)
ELISA plate: purchased from Nunc, Inc.

### Preparation of immunoprecipitates with anti-Rho-kinase peptide antibody

Extract fractions of the bovine brain were prepared as described below. The gray matter was removed from the bovine brain, cut finely with scissors, and then suspended in IP buffer. The suspension was homogenized with a nonstick homogenizer to destroy the tissue, and the homogenate was centrifuged at 15 K rpm for 15 minutes. The supernatant was collected, and further centrifuged at 35 K rmp for 60 minutes to provide the brain extract fraction (tissue homogenate).

Subsequently, one mg of the brain extract fraction was diluted with IP buffer to reach one ml, and 0.1 µg (0.5 µl) of the anti-Rho-kinase peptide polyclonal antibody was added to the dilution. The mixture was gently shaken at 4 °C for one and half hours, and after adding 15 µl of Protein G PLUS-agarose (SC-2002, Santa Cruz) thereto, the mixture was further gently shaken at 4 °C for one and half hours. The resultant mixture was centrifuged at 10,000 rpm for 30 seconds in a refrigerated centrifuge, and the supernatant was removed. The precipitated agarose beads were washed three times with one ml of IP buffer, and twice with K buffer. The whole amount of the resultant material was used in one reaction tube.
IP buffer: 20mM Tris-Cl (pH7.5), 150mM NaCl, 20mM NaF, 1mM EDTA, 1mM EGTA, and 0.2mM PMSF;
K buffer: 50mM Tris-Cl (pH7.5), 150mM NaCl, and 5mM MgCl₂.

In order to check if the Rho-kinase activity is dependent on the dose of anti-Rho-kinase antibody, the Rho-kinase activity in the immunoprecipitates formed with various doses of the antibody was determined using Histone HF2A as a substrate.

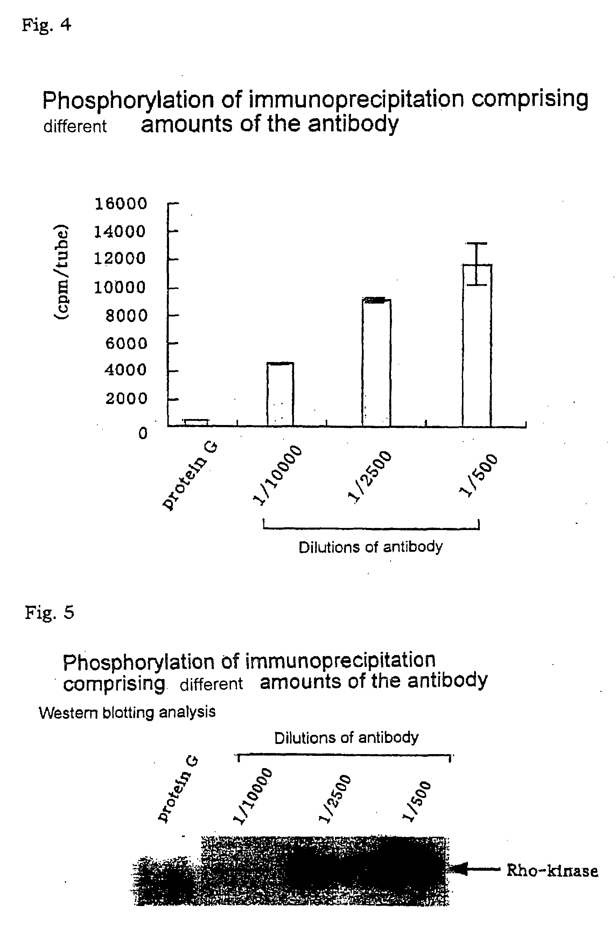

One mg of the bovine brain extract fraction was immunoprecipitated with 1/500 dilution (2 µg), 1/2500 dilution (0.4 µg), 1/10000 dilution (0.1 µg) of the antibody, and then the phosphorylations of the immunoprecipitates were determined. The results showed that the phosphorylations increased in an antibody dose-dependent manner (Figure 4).

Additionally, the immunoprecipitate products formed with the 1/500 dilution, the 1/2500 dilution, and the 1/10000 dilution of the antibody were electrophoresed on SDS-PAGE, subjected to Western blotting, and stained with the anti-Rho-kinase antibody (monoclonal antibody derived from mice, purchased from Transduction Laboratory), thus detecting the band of Rho-kinase at around 160 kDa. The results showed that the antibody captured Rho-kinase in a dose-dependent manner (Figure 5).

### Example 4

### RhoA-dependency of Rho-kinase activity in immunoprecipitates with anti-Rho-kinase antibody

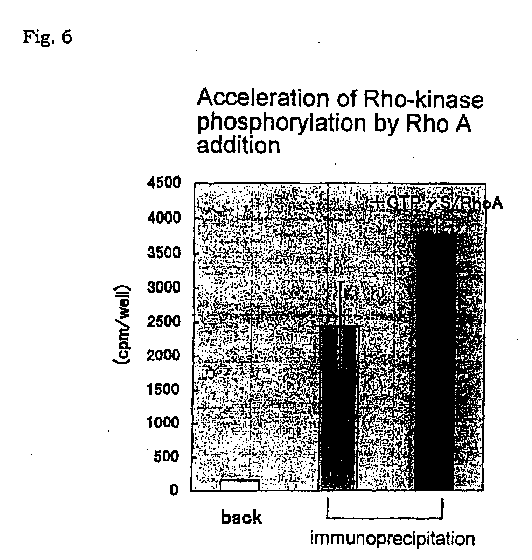

It has been known that the addition of activated RhoA induces acceleration of the phosphorylation of Rho-kinase by a factor of one and half to two (Matsui T. et al., *EMBO. J* 15, 2208-2216, 1996]. In fact, addition of GST-RhoA activated by GTPγS to the immunoprecipitate was found to accelerate the phosphorylation about 1.5 times compared with the addition of GST-RhoA inactivated by GDP (Figure 6).

### Example 5

### Preparation of plate immobilized with anti-Rho-kinase antibody

Fifty µl of the dilution of the rabbit anti-goat IgG/Fc (Nippon Chemi-Con Corp.) in PBS(-) was pipetted into Immunoplate II (Nunc, 442404, High binding capacity) or a fluorescence/luminescence solid assay plate (COSTAR, 3922 white). After allowed to stand for 1.5 hours at room temperature, the solution was removed, and 200 µl of blocking solution in which Block Ace (DAINIPPON PHARMACEUTICAL) UK-B25 was diluted four times with PBS(-)) was added into the plate, followed by stirring the solution therein. The plate was allowed to stand for additional 1.5 hours at room temperature, washed three times with 200 µl of PBST (0.05% Tween20/PBS(-)), and added with a 0.1 µg/50 µl solution of anti-Rho-kinase antibody in PBS(-). After allowed to stand for additional 1.5 hours at room temperature, the plate was washed three times with 200 µl of PBST. Into the plate was placed 50 µl of 1.5 mg/ml bovine brain lysates, and the resultant plate was allowed to stand overnight at 4 °C. This was washed twice with 200 µl of PBST, and once with 200 µl of PBS(-) to provide an immobilized plate.

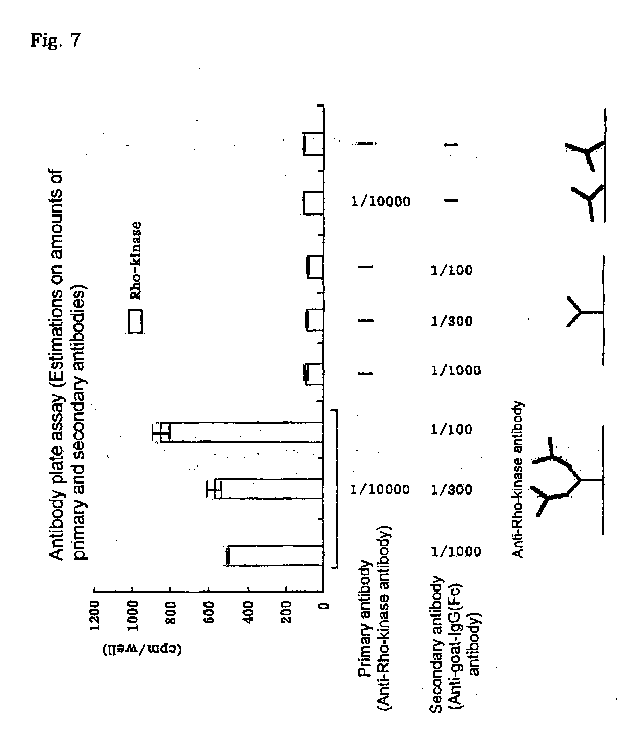

As shown above, the anti-Rho-kinase antibody was immobilized onto the ELISA plate via the secondary antibody recognizing its Fc fragment, and the phosphorylation was proceeded using Histone HF2A as a substrate. The secondary antibody was immobilized at 1/1,000, 1/300 and 1/100 dilutions onto the plate, and the anti-Rho-kinase antibody (primary antibody) was used at 1/10,000 dilution, which had allowed the detection of the activity in the immunoprecipitation. The results showed that a radioactivity of about 500-800 rpm was detected in the wells immobilized with the anti-Rho-kinase antibody, which activity was dependent on the dose of the antibody (Figure 7). On the other hand, the radioactivity in the wells immobilized with only the secondary antibody was about 100 rpm or less.

Additionally, the 1/10,000 dilution (0.1 µg) of the anti-Rho-kinase antibody only was immobilized onto the ELISA plate, and the phosphorylation was proceeded as shown above. The result showed that the radioactivity did not increase above the control level.

Those results as described above show that, in capturing the Rho-kinase activity with an anti-Rho-kinase antibody-immobilized ELISA plate, the immobilization of the antibody through the secondary antibody is better than the direct immobilization. Optimal conditions include 1/10,000 (0.1 µg) of the anti-Rho-kinase antibody, and 1/300 of the secondary antibody. It is understood that the failure to capture the activity by the direct immobilization of the antibody onto the plate would be caused by any steric hindrance at the antigen-recognition site of the primary antibody.

### Example 6

### Inhibition of the binding of Rho-kinase to anti-Rho-kinase antibody-immobilized plate with antigen peptides

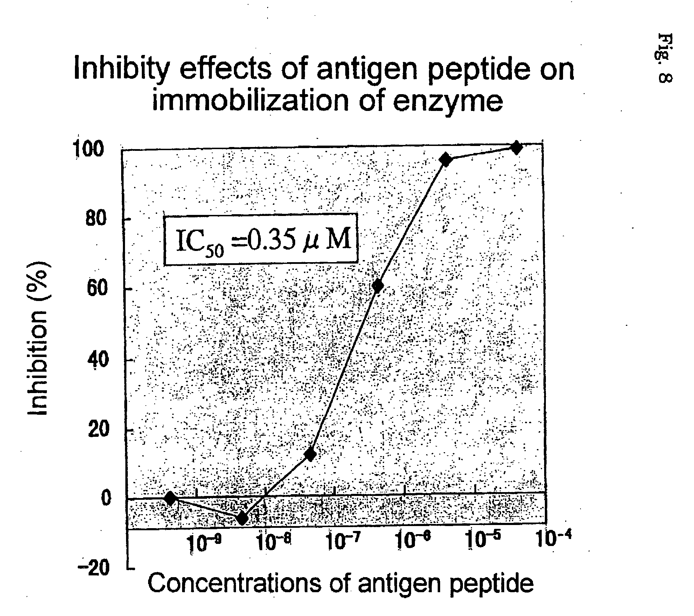

Various concentrations of the antigen peptide used to prepare the anti-Rho-kinase antibody were supplemented to the bovine brain extract, and then Rho-kinase in the extract was immobilized onto the plate under the optimal conditions as found in Example 5. The result showed that the peptide inhibited the immobilization of Rho-kinase in a concentration-dependent manner (Figure 8). Addition of 3.5 µM of the peptide inhibited almost 100% of the immobilization. IC₅₀ value was 0.35 µM.

### Example 7

### Examination of the effect of the Rho-kinase-immobilized plate using inhibitors of Rho-kinase

### I. Preparation of solution of test compound, and its reaction with the immobilized plate

A test compound was dissolved in a reaction solution composed of 20 mM Tris-Cl (pH 7.5), 10mM MgCl₂, 0.1 µM ATP 0.2 mg/ml, Histone HF2A, and 1.85 KBq [³³P] ATP/30 µl. Thirty µl of the solution was added to the Rho-kinase-immobilized plate prepared in Example 3 in order to start the reaction. The reaction time was four hours.

### II. Method for estimating activity/Method for determining phosphorylation

SPA Multiscreen method was used for the estimation of the plate.

Fasudil (HA1077, Asahi Kasei Corporation) and Y-27632 (Yoshitomi Pharmaceutical Industries) are known as inhibitors of Rho-kinase. The inhibitory activity of these inhibitors was determined based on the radioactivity showing the phosphorylation that was detected in the antibody plate assay system, and compared to those previously reported, so that the plate was estimated in terms of the usefulness in a screening system for discovering inhibitors. The inhibitory activities were 0.56 µM, and 0.35 µM in IC50 value, respectively. On the other hand, the value of chelerythrine was 11 µM.

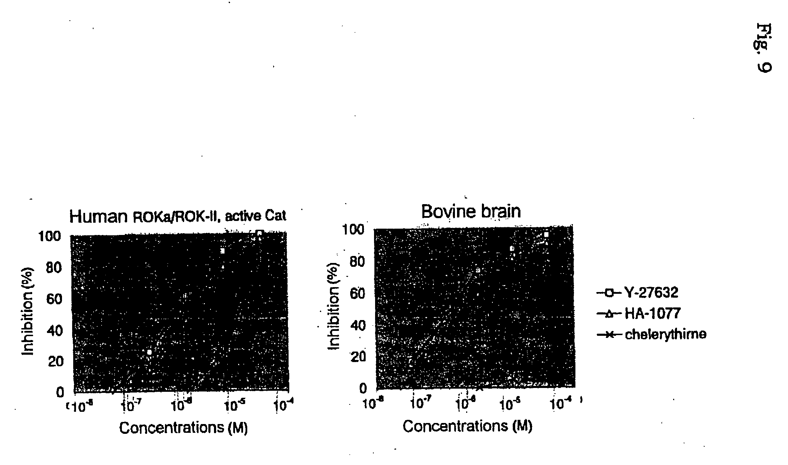

ROKa/ROK-II, active Cat (Upstate) was used to determine the inhibitory activities of the three inhibitors as described above under a similar condition for assay. IC50 values were 0.1.5 µM, 0.79 µM, and 11 µM, respectively, each of which was similar to the above value. The results are shown in Table 1 and Figure 9.

**Table 1**

| | IC50value | |
|---|---|---|
| | ROKa/ROK-II active Cat | bovine brain |
| HA-1077 | 1.5 | 0.56 |
| Y-27632 | 0.79 | 0.35 |
| chelerythirne | 11 | 20 |

Uehera M. et al., *Nature* 389, 990-994, 1997 describes that Fasudil and Y-27632 are 0.33 µM and 0.14 µM, respectively, in terms of Ki value (under the reaction condition herein, Ki = IC50). The IC50 values obtained in this example were not inconsistent with those described in the article.

The inconsistency between the values of the article obtained by isolating and purifying Rho-kinase and then determining the inhibitory activities of the inhibitors, and those obtained in the present screening method shows the usefulness of the present screening method. The fact has proved that the present method can screen efficiently enzymatic proteins such as Rho-kinase, which are unstable and difficult for their isolation in a large amount.

### INDUSTRIAL APPLICABILITY

The present invention provides a method of screening for activators or inhibitors of enzymatic proteins that are unstable and difficult to isolate, as well as screening instruments for the method. Since Rho-kinase is responsible for the smooth muscle contraction, the inhibitors obtainable by conducting the screening method of the present invention should exhibit an inhibitory activity of the contraction, and therefore be used as medicaments or prophylactics for cerebrovascular diseases associated with the contraction such as hypertension.

## Claims

1. A method of screening for an activator or an inhibitor of the protein function of a soluble protein or a potentially soluble protein, which comprises:
1) preparing a partial peptide comprising a part of the amino acid sequence of the protein, said partial peptide having the following properties that;
i) the partial peptide comprises an amino acid sequence characteristic of the protein and distinct from proteins belonging to the same family as the protein;
ii) the amino acid sequence comprises at least six amino acid residues, and is located in a highly hydrophilic region;
iii) the amino acid sequence has a functional group capable of binding to a carrier protein; and
iv) the protein is not inactivated by the reaction between the protein and an antibody raised using the partial peptide as an antigen;
2) preparing an antibody having an affinity of 10⁵/M or less, by use of the partial peptide as an antigen;
3) preparing a solid support immobilized with an antibody to the antibody (Ig) prepared in step 2;
4) immobilizing the antibody (Ig) prepared in step 2 on the solid support prepared in step 3;
5) applying a cell or tissue homogenate containing the protein to the solid support prepared in step 4 to immobilize the protein thereon;
6) reacting a solution of a test substance with the solid support immobilized with the protein as prepared in step 5; and
7) measuring the activity of the protein after completion of the reaction, so as to determine the effect of the test substance on the protein function.

2. The method of claim 1, wherein the soluble protein or the potentially soluble protein is a solubilized enzyme.

3. The method of claim 2, wherein the solubilized enzyme is a serine/threonine kinase.

4. The method of claim 1, wherein the protein function of the soluble protein or the potentially soluble protein is determined by the incorporation of ³²P or ³³P into a substrate, which is estimated using radioactivity of the phosphorus or binding activity of an antibody to a phosphorylated substrate.

5. The method of claim 4, wherein the protein function is determined by the effect of the test substance, which is estimated using:
a) SPA (Scintillation Proximity Assay) method,
b) Multiscreen method, or
c) a filter-spot method.

6. The method of claim 3, wherein the serine/threonine kinase is Rho-kinase.

7. The method of claim 6, wherein Rho-kinase is from bovine, mouse, or rat.

8. The method of any one of claims 1 to 7, wherein the tissue homogenate is from bovine cerebral gray matter.

9. The method of any one of claims 1 to 8, wherein the soluble protein is Rho-kinase, and the partial peptide comprises 20 amino acid residues resided at the C-terminal part of Rho-kinase.

10. The method of any one of claims 1 to 9, wherein the partial peptide comprises the 20 amino acid residues of IQQNQSIRRPSRQLAPNKPS.

11. An activator or an inhibitor of the protein function, which is obtainable by conducting the method of any one of claims 1 to 10.

12. The activator or the inhibitor of claim 11, wherein the protein is Rho-kinase.

13. The activator or the inhibitor of claim 12, wherein the protein is bovine Rho-kinase.

14. A pharmaceutical composition, which comprises the activator or the inhibitor of any one of claims 11 to 13 as an active ingredient in admixture with a pharmaceutically acceptable excipient.

15. A pharmaceutical composition for inhibiting phosphorylation, which comprises the inhibitor of Rho-kinase of claim 12 as an active ingredient in admixture with a pharmaceutically acceptable excipient.

16. A pharmaceutical composition for inhibiting smooth muscle contraction, which comprises the inhibitor of Rho-kinase of claim 12 as an active ingredient in admixture with a pharmaceutically acceptable excipient.

17. A pharmaceutical composition for treating hypertension, cardiac angina, or cerebrovascular spasm, which comprises the inhibitor of Rho-kinase of claim 12 as an active ingredient in admixture with a pharmaceutically acceptable excipient.

18. A method of treating hypertension, cardiac angina, or cerebrovascular spasm, which comprises administering a patient having or at risk of developing one of the diseases an effective amount of the composition of claim 17.

19. A pharmaceutical composition for promoting phosphorylation, which comprises the activator of Rho-kinase of claim 12 as an active ingredient in admixture with a pharmaceutically acceptable excipient.

20. A pharmaceutical composition for inducing smooth muscle contraction, which comprises the activator of Rho-kinase of claim 12 as an active ingredient in admixture with a pharmaceutically acceptable excipient.

21. A method of purifying a protein, which comprises:
1) preparing a partial peptide comprising a part of the amino acid sequence of the protein, said partial peptide having the following properties that;
i) the partial peptide comprises an amino acid sequence characteristic of the protein and distinct from proteins belonging to the same family as the protein;
ii) the amino acid sequence comprises at least six amino acid residues, and is located in a highly hydrophilic region;
iii) the amino acid sequence has a functional group capable of binding to a carrier protein; and
iv) the protein is not inactivated by the reaction between the protein and an antibody raised using the partial peptide as an antigen;
2) preparing an antibody having an affinity of 10⁵/M or less (Ig), by use of the partial peptide as an antigen;
3) preparing a solid support immobilized with an antibody to the antibody (Ig) prepared in step 2;
4) immobilizing the antibody (Ig) prepared in step 2 on the solid support prepared in step 3;
5) applying a cell or tissue homogenate containing the protein to the solid support prepared in step 4 to immobilize the protein thereon; and
6) releasing and recovering the protein from the solid support.

22. The method of claim 21, wherein the protein is an unstable protein, a soluble protein or a potentially soluble protein, preferably Rho-kinase.

23. The method of claim 21, wherein the protein is Rho-kinase derived from bovine, mouse, or rat.

24. A solid support useful for a purification system of a protein or a screening system of a soluble protein or a potentially soluble protein, which is prepared by a process comprising:
1) preparing a partial peptide comprising a part of the amino acid sequence of the protein, said partial peptide having the following properties that;
i) the partial peptide comprises an amino acid sequence characteristic of the protein and distinct from proteins belonging to the same family as the protein;
ii) the amino acid sequence comprises at least six amino acid residues, and is located in a highly hydrophilic region;
iii) the amino acid sequence has a functional group capable of binding to a carrier protein; and
iv) the protein is not inactivated by the reaction between the protein and an antibody raised using the partial peptide as an antigen; protein;
2) preparing an antibody having an affinity of 10⁵/M or less (Ig), by use of the partial peptide as an antigen;
3) preparing a solid support immobilized with an antibody to the antibody (Ig) prepared in step 2; and
4) immobilizing the antibody (Ig) prepared in step 2 on the solid support prepared in step 3.

25. The solid support of claim 24, wherein the protein is Rho-kinase.

26. A solid support immobilized with a soluble protein or a potentially soluble protein useful for a screening system, which is prepared by a process comprising:
1) preparing a partial peptide comprising a part of the amino acid sequence of the protein, said partial peptide having the following properties that;
i) the partial peptide comprises an amino acid sequence characteristic of the protein and distinct from proteins belonging to the same family as the protein;
ii) the amino acid sequence comprises at least six amino acid residues, and is located in a highly hydrophilic region;
iii) the amino acid sequence has a functional group capable of binding to a carrier protein; and
iv) the protein is not inactivated by the reaction between the protein and an antibody raised using the partial peptide as an antigen;
2) preparing an antibody having an affinity of 10⁵/M or less (Ig), by use of the partial peptide as an antigen;
3) preparing a solid support immobilized with an antibody to the antibody (Ig) prepared in step 2;
4) immobilizing the antibody (Ig) prepared in step 2 on the solid support prepared in step 3; and
5) applying a cell or tissue homogenate containing the protein to the solid support prepared in step 4 to immobilize the protein thereon.

27. The solid support of claim 26, wherein the soluble protein or the potentially soluble protein is Rho-kinase, and the support is in the form of plate.

28. The solid support of claim 27, wherein the tissue homogenate is from the bovine cerebral gray matter, or from the rat brain.

29. The solid support of claim 27 or 28, wherein Rho-kinase is from bovine.
